(19) Europäisches Patentamt
European Patent Office
Office européen des brevets

(11) **EP 1 121 091 B1**

(12) **EUROPÄISCHE PATENTSCHRIFT**

(45) Veröffentlichungstag und Bekanntmachung des Hinweises auf die Patenterteilung:
**21.04.2004 Patentblatt 2004/17**

(21) Anmeldenummer: **99970313.5**

(22) Anmeldetag: **05.10.1999**

(51) Int Cl.[7]: **A61K 7/13**

(86) Internationale Anmeldenummer:
**PCT/EP1999/007368**

(87) Internationale Veröffentlichungsnummer:
**WO 2000/021497 (20.04.2000 Gazette 2000/16)**

(54) **FÄRBEMITTEL MIT ENZYMEN**

COLOURING AGENTS WITH ENZYMES

COLORANTS COMPRENANT DES ENZYMES

(84) Benannte Vertragsstaaten:
**AT BE CH CY DE DK ES FI FR GB GR IE IT LI LU MC NL PT SE**

(30) Priorität: **14.10.1998 DE 19847276**

(43) Veröffentlichungstag der Anmeldung:
**08.08.2001 Patentblatt 2001/32**

(73) Patentinhaber: **Henkel Kommanditgesellschaft auf Aktien**
**40589 Düsseldorf-Holthausen (DE)**

(72) Erfinder:
- **SÄTTLER, Andrea**
  **D-40225 Düsseldorf (DE)**
- **KLEEN, Astrid**
  **D-40699 Erkrath (DE)**
- **WEISS, Albrecht**
  **D-40764 Langenfeld (DE)**
- **ROSE, David**
  **D-40723 Hilden (DE)**

(56) Entgegenhaltungen:
**DE-A- 19 545 729** **DE-A- 19 713 852**
**FR-A- 2 768 618**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Mittel zur Färbung von Keratinfasern, die ein spezielles Enzymsystem enthalten, deren Verwendung sowie entsprechende Verfahren zum Färben keratinischer Fasern.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehrerer Kuppler-komponenten die eigentlichen Farbstoffe aus.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt.

Als Entwicklerkomponenten werden üblicherweise primäre aromatische Amine mit einer weiteren, in para- oder ortho-Position befindlichen, freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-aminopyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2'-hydroxyethyl)-N,N'-bis(4'-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Methylresorcin, 5-Methylresorcin und 2-Methyl-4-chlor-5-aminophenol.

Die oxidative Entwicklung der Färbung kann grundsätzlich mit Luftsauerstoff erfolgen. Bevorzugt wird jedoch ein chemisches Oxidationsmittel eingesetzt. Als Oxidationsmittel kommen Persulfate, Chlorite und insbesondere Wasserstoffperoxid oder dessen Anlagerungsprodukte an Harnstoff, Melamin sowie Natriumborat in Frage. Üblicherweise wird eine etwa 2-9%ige wäßrige Wasserstoffperoxidlösung eingesetzt. Unter der Einwirkung derart hoher Oxidationsmittelkonzentrationen können die keratinischen Fasern, insbesondere wenn sie bereits dauergewellt oder gebleicht sind, geschädigt werden, und in seltenen Fällen können durch diese hohen Konzentrationen auch Hautirritationen auftreten.

Ein wesentlicher Lösungsansatz zu dieser Problematik geht von der Reduzierung der Oxidationsmittelkonzentration aus. Es wurde daher in der Vergangenheit einerseits nach Farbstoffvorprodukten gesucht, die aufgrund ihrer chemischen Struktur bereits durch geringere Mengen Wasserstoffperoxid oder durch Luftsauerstoff oxidiert werden können. Andererseits wurde die Verwendung von Enzymen als Biokatalysatoren vorgeschlagen, die den erwünschten Oxidationsprozess mit sehr wenig oder ganz ohne Wasserstoffperoxid nur in der Anwesenheit von Luftsauerstoff katalysieren können.

In der deutschen Offenlegungsschrift DE-OS-2 155 390 wird ein enzymaktiviertes, oxidatives Haarfärbeverfahren beschrieben, bei dem geringe Mengen $H_2O_2$ in Kombination mit einem Peroxidase-Enzym eingesetzt werden. Auch in der EP-A1-0 310 675 werden enzymatische Haarbehandlungsmittel offenbart, die mindestens eine Dielektronenreduzierende Oxidase, die Sauerstoff als Akzeptor nutzt, enthalten. In der EP-B1-0 548 620 werden enzymatische Haarfärbemittel beschrieben, bei denen die Oxidation der Farbstoffvorprodukte durch Einsatz einer Peroxidase katalysiert wird. Schließlich werden in der EP-A2-0 795 313 enzymatische Haarfärbemittel beschrieben, die ein Sauerstoff-Oxidoreduktase/Substrat-System und eine Peroxidase sowie als Kupplerkomponenten zwingend ein m-Phenylendiaminderivat enthalten. Alle diese Färbemittel können aber bezüglich der erzielbaren Färbeleistung (Intensität, Nuance, Glanz, Echtheitseigenschaften) noch nicht vollständig überzeugen.

Die Technik der leicht-oxidierbaren Farbstoffvorprodukte hat ebenso wie die bislang beschriebene enzymatische Farbentwicklung den Nachteil, daß im Vergleich zu den herkömmlichen Verfahren insbesondere bezüglich der Intensität, des Glanzes und der Echtheitseigenschaften der Färbungen schlechtere Ergebnisse erzielt werden.

Die vorliegende Erfindung geht daher von der Aufgabe aus, Färbemittel für keratinische Fasern zur Verfügung zu stellen, die eine schonende Faserbehandlung ermöglichen und gleichzeitig eine hervorragende Färbeleistung gewährleisten.

Es wurde nun überraschenderweise gefunden, daß Färbemittel mit guter Färbeleistung und deutlichen Vorteilen im Bereich der Schonung und Pflege der Fasern sowie der Haut erhalten werden, wenn sie mindestens ein Farbstoffvorprodukt, ein Oxidase-System auf Basis des Cholins sowie mindestens eine Peroxidase enthalten.

Ein erster Gegenstand der vorliegenden Anmeldung sind daher Mittel zum Färben keratinischer Fasern, enthaltend mindestens ein Farbstoffvorprodukt, Cholin-Oxidase in Kombination mit Cholin als Substrat sowie mindestens eine Peroxidase, wobei die Cholin-Oxidase in Mengen von 1-50 000U pro 100g Färbezubereitung, das Substrat Cholin in Mengen von 1-5 Gew.-%, bezogen auf die gesamte Färbezubereitung, und die Peroxidase in einer Menge von 1-100 000U, bezogen auf 100g Färbezubereitung, eingesetzt werden.

Unter Keratinfasern sind dabei Pelze, Wolle, Federn und insbesondere menschliche Haare zu verstehen.

Unter einem Oxidase-System auf Basis des Cholins ist erfindungsgemäß Cholin-Oxidase in Kombination mit Cholin als Substrat zu verstehen. Die Oxidation von Cholin durch Cholin-Oxidase verläuft nach folgendem Reaktionsschema:

$$\text{Cholin} + O_2 \xrightarrow{\text{Cholin-Oxidase}} \text{Betainaldehyd} + H_2O_2 \xrightarrow[+ O_2 + H_2O]{\text{Cholin-Oxidase}} \text{Betain} + H_2O_2$$

Cholin wird im Sinne der vorliegenden Erfindung bevorzugt als Salz mit einer physiologisch verträglichen organischen oder anorganischen Säure eingesetzt. Beispiele für solche Salze sind das Chlorid, das Bromid, das Iodid, das Citrat, das Hydrogentartrat, das Hydrogencarbonat, das Methylsulfat sowie das p-Toluolsulfonat. Besonders bevorzugt ist Cholinchlorid.

Cholin-Oxidase (EC 1.1.3.17) kann aus vielen Quellen gewonnen werden; so wurde beispielsweise ein Vorkommen in menschlichen Zellen nachgewiesen. Cholin-Oxidase, die von *Alcaligenes species* und *Arthrobacter globiformis* produziert wird, ist erfindungsgemäß besonders bevorzugt. Cholin-Oxidasen sind im Handel erhältlich und werden beispielsweise von der Firma Sigma angeboten. Die Aktivität der Cholin-Oxidase ist erfindungsgemäß derart definiert, daß eine Einheit [1U] Cholin-Oxidase die Oxidation von 1µmol Cholin zu Betainaldehyd innerhalb einer Minute bei pH 8,0 und 37°C katalysiert, wobei 1µmol $H_2O_2$ gebildet wird (entsprechend der Definition der Handelsprodukte der Firma Sigma).

Cholin-Oxidase wird in den erfindungsgemäßen Mitteln in Mengen von 1-50 000U pro 100g Färbezubereitung eingesetzt. Bevorzugt sind Mengen von 1-10 000U pro 100g Färbezubereitung, ganz besonders bevorzugt ist der Bereich von 400-5 000U pro 100g Färbezubereitung.

Das Substrat Cholin wird erfindungsgemäß in Mengen von 1-5 Gew.-%, bezogen auf die gesamte Färbezubereitung, eingesetzt.

Neben dem Oxidase-System auf Basis des Cholins enthalten die erfindungsgemäßen Mittel zusätzlich noch eine Peroxidase (EC 1.11.1.7). Die Peroxidasen können aus Tieren, Pflanzen oder Pilzen gewonnen werden. Pflanzliche Peroxidasen und Peroxidase auf der Basis von Pilzen können erfindungsgemäß bevorzugt sein, Sojabohnen-Peroxidase ist ganz besonders bevorzugt.

In einer weiteren Ausführungsform der vorliegenden Erfindung kann es bevorzugt sein, Peroxidasen mit einem möglichst geringen Katalase-Gehalt, wie beispielsweise Meerrettich-Peroxidase, zu verwenden.

Die Aktivität der Peroxidase ist erfindungsgemäß derart definiert, daß eine Einheit [1U] Peroxidase 1,0 mg Purpurogallin aus Pyrogallol innerhalb von 20sec bei pH 6,0 und 20°C bildet (entsprechend der Definition der Handelsprodukte der Firma Sigma).

Die Peroxidase wird erfindungsgemäß in einer Menge von 1-100 000U eingesetzt, bevorzugt sind Mengen von 1-10 000U, ganz besonders bevorzugt ist eine Menge von 1-500U. Die angegebenen Mengen beziehen sich jeweils auf 100g Färbezubereitung.

Die erfindungsgemäßen Mittel zeichnen sich durch ihre haar- und hautschonende Wirkung aus. Die erfindungsgemäß behandelten Haare weisen eine erhöhte Haarglätte sowie eine höhere Reißfestigkeit und eine geringere Porosität auf als Haare, die mit herkömmlichen Färbetechniken gefärbt wurden.

Gemäß einer ersten Ausführungsform der vorliegenden Erfindung kann das Farbstoffvorprodukt ein Oxidationsfarbstoffvorprodukt vom Typ Entwickler sein. Es können aber auch mehrere Entwickler in den erfindungsgemäßen Mitteln eingesetzt werden.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 1,8-Bis-(2,5-Diaminophenoxy)-3,6-dioxaoctan, 4-Amino-2-(2-hydroxyethoxy)-phenol sowie 4,5-Diaminopyrazol-Derivate nach EP 0 740 741 bzw. WO 94/08970 wie z.B. 4,5-Di-amino-1-(2'-hydroxyethyl)-pyrazol.

Besonders bevorzugte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 4,5-Diamino-1-(2-hydroxyethyl)-pyrazol, 2-Aminomethyl-4-aminophenol, N,N-Bis-(2-hydroxyethyl)-p-phenylendiamin, 1,8-Bis-(2,5-Diaminophenoxy)-3,6-dioxa-octan, Bis-(2-hydroxy-5-aminophenyl)-methan und o-Aminophenol.

Ganz besonders bevorzugte Entwicklerkomponenten sind 1-Methyl-2,5-diaminobenzol, 4-Amino-2-aminomethylphenol, p-Aminophenol, 4-Hydroxy-2,5,6-triaminopyrimidin, 1-(2'-Hydroxyethyl)-2,5-diaminobenzol sowie N, N-Bis-(2-hydroxyethyl)-1,4-diaminobenzol.

Zur Nuancierung der erzielbaren Farbtöne können die erfindungsgemäßen Mittel weiterhin noch eine oder mehrere Kupplerkomponenten enthalten. Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminaphenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dimethoxy-3,5-diaminopyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Besonders bevorzugte Kupplerkomponenten im Sinne der Erfindung sind 1-Naphthol, 1,5-, 2,7- und 1,7-Dihydroxynaphthalin, 5-Amino-2-methylphenol, Resorcin, 1,3-Bis-(2,4-diaminophenoxy)-propan, 2,4-Dichlor-3-aminophenol, 4-Chlorresorcin, 2-Amino-3-hydroxypyridin, 2,6-Dimethoxy-3,5-diaminopyridin, 2-Chlor-6-methyl-3-aminophenol, 2-Methyl-4-chlor-5-amino-phenol, 2-Methylresorcin, 5-Methylresorcin, 2,5-Dimethylresorcin, 2,6-Bis-(2-hydroxyethylamino)-toluol, 2,6-Dihydroxy-3,4-diaminopyridin, 3-Amino-2-methylamino-6-methoxypyridin, 2,4-Diaminophenoxyethanol, 1-Methoxy-2-amino-4-(2-hydroxyethylamino)-benzol, 6-Methyl-1,2,3,4-tetrahydro-chinoxalin, 3,4-Methylendioxyanilin, m-Aminophenol, o-Aminophenol und 2-Chlorresorcin.

Ganz besonders bevorzugte Kupplerkomponenten sind 2,4-Diaminophenoxyethanol, 2-Chlor-6-methyl-3-aminophenol, 5-Amino-2-methylphenol, 1,3-Bis-(2,4-diaminophenoxy)-propan, 2-Methylresorcin sowie 1-Methoxy-2-amino-4-(2-hydroxyethylamino)-benzol.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die folgenden Entwickler/Kupplerkombinationen haben sich als besonders geeignet im Sinne der Erfindung erwiesen:

- 4-Aminophenol/5-Amino-2-methylphenol
- 1-Methyl-2,5-diaminobenzol/2,4-Diaminophenoxyethanol
- 4-Amino-2-aminomethylphenol/2-Chlor-6-methyl-3-aminophenol
- 4-Amino-2-aminomethylphenol/2,4-Diaminophenoxyethanol
- 1-Methyl-2,5-diaminobenzol/5-Amino-2-methylphenol
- 1-Methyl-2,5-diaminobenzol/1,3-Bis-(2,4-diaminophenoxy)-propan
- 4-Hydroxy-2,5,6-triaminopyrimidin/2-Methylresorcin
- 1-(β-Hydroxyethyl)-2,5-diaminobenzol/2,4-Diaminophenoxyethanol
- N,N-Bis-(β-hydroxyethyl)-1,4-diaminobenzol/1-Methoxy-2-amino-4-(2-hydroxyethylamino)-benzol.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa gleichen molaren Mengen zueinander eingesetzt. Wenn sich auch der äqui-molare Einsatz als zweckmäßig erwiesen hat, so ist ein gewisser Überschuß einzelner Oxidationsfarbstoffvorprodukte nicht nachteilig, so daß Entwicklerkomponenten und Kuppler-komponenten in einem Mol-Verhältnis von 1:0,5 bis 1:3, insbesondere 1:1 bis 1:2, enthalten sein können.

Gemäß einer zweiten bevorzugten Ausführungsform der vorliegenden Erfindung kann das Farbstoffvorprodukt ein Derivat des Indolins sein. Bevorzugte Beispiele sind die Derivate des 5,6-Dihydroxyindolins der Formel (Ia),

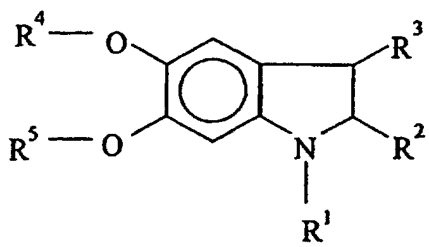

(Ia)

in der unabhängig voneinander $R^1$ steht für Wasserstoff, eine $C_1$- bis $C_4$-Alkylgruppe oder eine $C_1$- bis $C_4$-Hydroxyalkylgruppe, $R^2$ steht für Wasserstoff oder eine -COOH-Gruppe, wobei die -COOH-Gruppe auch als Salz mit einem physiologisch verträglichen Kation vorliegen kann, $R^3$ steht für Wasserstoff oder eine $C_1$- bis $C_4$-Alkylgruppe, $R^4$ steht für Wasserstoff, eine $C_1$- bis $C_4$-Alkylgruppe oder eine Gruppe -CO-$R^6$, in der $R^6$ steht für eine $C_1$- bis $C_4$-Alkylgruppe, und $R^5$ steht für eine der unter $R^4$ genannten Gruppen, oder ein physiologisch verträgliches Salz dieser Verbindungen mit einer organischen oder anorganischen Säure.

In einer dritten bevorzugten Ausführungsform der vorliegenden Erfindung kann das Farbstoffvorprodukt ein Derivat des Indols sein. Bevorzugte Beispiele sind die Derivate des 5,6-Dihydroxyindols der Formel (Ib),

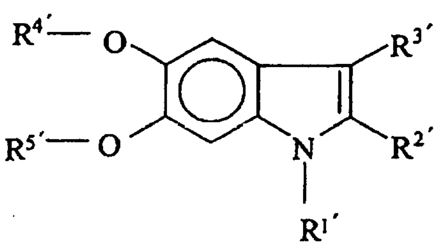

(Ib)

in der unabhängig voneinander $R^{1'}$ steht für Wasserstoff, eine $C_1$- bis $C_4$-Alkylgruppe oder eine $C_1$- bis $C_4$-Hydroxyalkylgruppe, $R^{2'}$ steht für Wasserstoff oder eine -COOH-Gruppe, wobei die -COOH-Gruppe auch als Salz mit einem physiologisch verträglichen Kation vorliegen kann, $R^{3'}$ steht für Wasserstoff oder eine $C_1$- bis $C_4$-Alkylgruppe, $R^{4'}$ steht für Wasserstoff, eine $C_1$- bis $C_4$-Alkylgruppe oder eine Gruppe -CO-$R^{6'}$, in der $R^{6'}$ steht für eine $C_1$- bis $C_4$-Alkylgruppe, und $R^{5'}$ steht für eine der unter $R^{4'}$ genannten Gruppen, oder ein physiologisch verträgliches Salz dieser Verbindungen mit einer organischen oder anorganischen Säure.

Besonders bevorzugte Derivate des Indolins sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin. Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbon-säure, 6-Hydroxyindol, 6-Aminoindol und 4-Aminoindol.

Ganz besonders bevorzugt sind 5,6-Dihydroxyindolin sowie 5,6-Dihydroxyindol.

In einer ersten Variante der oben beschrieben Ausführungsformen werden die Mittel derart formuliert, daß sie als Farbstoffvorprodukte nur Indol- und/oder Indolinderivate enthalten und frei sind von üblichen Oxidationsfarbstoffvorprodukten vom Entwickler/Kuppler-Typ.

In einer zweiten Variante der oben beschriebenen Ausführungsformen können die erfindungsgemäßen Mittel neben den Indol- und/oder Indolinderivaten auch noch übliche Oxidationsfarbstoffvorprodukte vom Entwickler/Kuppler-Typ enthalten. Im Rahmen dieser Variante kann es erfindungsgemäß bevorzugt sein, das Indolin- oder das Indolderivat in Kombination mit einer oder mehreren Kuppler-komponenten in Haarfärbemitteln einzusetzen. Es sei an dieser Stelle ausdrücklich auf die oben als bevorzugt genannten Kupplerkomponenten verwiesen.

In einer weiteren Ausführungsform kann es erfindungsgemäß bevorzugt sein, das Indolinoder Indolderivat in Haarfärbemitteln in Kombination mit mindestens einer Aminosäure oder einem Oligopeptid einzusetzen. Die Aminosäure ist vorteilhafterweise eine $\alpha$-Aminosäure ist; ganz besonders bevorzugte $\alpha$-Aminosäuren sind Arginin, Ornithin, Lysin und Histidin.

In einer weiteren bevorzugten Ausführungsform enthalten die erfindungsgemäßen Haarfärbemittel zur weiteren Modifizierung der Farbnuancen neben den Farbstoffvorprodukten zusätzlich direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Farbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Bevorzugt sind Haarfärbemittel auf Basis des Cholin-Oxidasesystems, die einen pH-Wert von 7 bis 10, insbesondere einen pH-Wert von 8 bis 9 aufweisen. Es wurde im Rahmen der vorliegenden Erfindung gefunden, daß besonders intensive und glänzende Färbungen erzielt werden, wenn das Mittel einen pH-Wert von etwa 8,3 aufweist. Es kann bevorzugt sein, daß der pH-Wert des Haarfärbemittels mit einem Tris(hydroxymethyl)-aminomethan/Kaliumchlorid-Puffer-system eingestellt wird.

Neben dem erfindungsgemäßen Cholin-Oxidasesystem können die erfindungsgemäßen Färbemittel auch weitere Oxidasen mit ihren jeweiligen Substraten enthalten. Beispiele sind Glucose-Oxidase, Alkohol-Oxidase, Pyruvat-Oxidase, Oxalat-Oxidase, Cholesterin-Oxidase, Uricase, Lactat-Oxidase, Xanthin-Oxidase, Pyranose-Oxidase, Glycerin-Oxidase sowie Galactose-Oxidase. Besonders bevorzugt sind Mittel, die neben dem Cholin-Oxidasesystem weiterhin Glucose-Oxidase und/oder Xanthin-Oxidase sowie deren jeweilige Substrate enthalten.

Zur Herstellung der erfindungsgemäßen Färbemittel können die Farbstoffvorprodukte in einen geeigneten wasserhaltigen Träger eingearbeitet werden. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. Der Fachmann kann einen eventuellen Einfluß der verschiedenen Tenside auf die Aktivität des erfindungsgemäßen Enzymsystems gegebenenfalls durch einfache Vorversuche überprüfen.

In einer bevorzugten Ausführungsform der vorliegenden Erfindung wird in den Mitteln zur Färbung keratinischer Fasern eine Kombination aus anionischen und nichtionischen Tensiden oder eine Kombination aus anionischen und amphoteren Tensiden eingesetzt.

Es hat sich aber in Einzelfällen als vorteilhaft erwiesen, die Tenside aus amphoteren oder nichtionischen Tensiden auszuwählen, da diese in der Regel den erfindungsgemäßen Färbeprozeß weniger beeinflussen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2-CH_2O)_x-CH_2-COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,

- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R\text{-}O(CH_2\text{-}CH_2O)_x\text{-}SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Unter ampholytischen Tensiden werden solche oberflächenaktiven Verbindungen verstanden, die außer einer $C_{8-18}$-Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12-22}$-Fettsäuremono- und -diester von Anlagerungsprodukten von 1 bis 30 Mol Ethylenoxid an Glycerin,
- $C_{8-22}$-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide sowie
- Aminoxide.

Alkylpolyglykoside gemäß der Formel (II)

$$R^6—O—(Z)_X \qquad (II),$$

in der $R^6$ steht für einen Alkylrest mit 8 bis 22 Kohlenstoffatomen, Z für einen Monooder Oligosaccharid und x für eine Zahl von 1,1 bis 5, sind besonders bevorzugte nichtionogene Tenside. Diese Verbindungen sind durch die folgenden Parameter gekennzeichnet.

Der Alkylrest $R^6$ enthält 8 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können lediglich einen bestimmten Alkylrest $R^6$ enthalten. Üblicherweise werden diese Verbindungen aber ausgehend von natürlichen Fetten und Ölen oder Mineralölen hergestellt. In diesem Fall liegen als Alkylreste $R^6$ Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^6$

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,3 bis 2 sind bevorzugt. Ganz besonders bevorzugt sind Alkylglykoside, bei denen x 1,4 bis 1,6 beträgt.

Erfindungsgemäß weiterhin bevorzugt sind Aminoxide der allgemeine Formel (VI)

$$R^7R^8R^9N \rightarrow O \qquad \text{(III),}$$

wobei $R^7$ für eine verzweigte oder unverzweigte $C_8$- bis $C_{18}$-Alkylkette steht und $R^8$ und $R^9$ unabhängig voneinander stehen für eine $C_1$- bis $C_3$-Alkylgruppe oder eine $C_1$- bis $C_3$-Hydroxyalkylgruppe.

Ein erfindungsgemäßes Aminoxid ist ferner das von der Firma Tego® Cosmetics vertriebene Aminoxid WS 35, wobei $R^8$ und $R^9$ für Methylgruppen stehen und $R^7$ ein Kokosacylamidopropylrest ist.

Als zwitterionische Tenside werden solche oberflächenaktiven Verbindungen bezeichnet, die im Molekül mindestens eine quartäre Ammoniumgruppe und mindestens eine -$COO^{(-)}$-oder -$SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Beispiele für die in den erfindungsgemäßen Haarfärbemitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammo-niumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex® vertriebenen Methyl-hydroxyalkyldialkoyloxyalkyl-ammoniummethosulfate sowie die unter dem Warenzeichen Dehyquart® vertriebenen Produkte wie Dehyquart AU-46.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyi Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid

unter Verwendung von Alkalimetallen, Alkalimetallhydroxidcn oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quatemären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäu-reanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecithin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine, Aminosäuren
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, $N_2O$, Dimethylether, $CO_2$ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Zweckmäßigerweise wird die Enzymzubereitung unmittelbar vor dem Haarefärben mit der Zubereitung aus den Farbstoffvorprodukten vermischt. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Um den Färbevorgang zu beschleunigen, kann es erfindungsgemäß bevorzugt sein, das Oxidase-System (oder ggbf. dessen wäßrige Lösung)vor Mischung mit der Färbezubereitung 30min bei 37°C, beispielsweise in einer Schüttelhaube, vorzuinkubieren. Gegebenfalls kann das System vor der Inkubation separat auf den gewüschten pH-Wert eingestellt werden. Der Vorinkubationsansatz wird anschließend in die Färbecreme eingearbeitet. Abschließend wird die Peroxidase zugemischt.

In einer weiteren Ausführungsform der vorliegenden Erfindung kann es bevorzugt sein, die Enzymzubereitung frei von Antioxidantien und/oder Komplexbildner zu formulieren, da diese die Wirkung der Enzyme blockieren können.

Die nachfolgenden Beispiele sollen den Gegenstand der Erfindung näher erläutern.

**Ausführungsbeispiele**

***Beispiel 1***

***Färbungen mit dem erfindungsgemäßen Cholin-Oxidasesystem***

1 Herstellung der Färbecreme

*(a) Creme-Grundlage (Teilmischung A)*

| | |
|---|---|
| Hydrenol® D[1] | 8,50g |
| Lorol® techn.[2] | 2,00g |
| Eumulgin® B2[3] | 0,75g |
| Texapon® NSO[4] | 20,00g |
| Dehyton® K[5] | 12,50g |
| Wasser | 30,00g |

[1] $C_{16\text{-}18}$-Fettalkohol (INCI-Bezeichnung: Cetearyl alcohol) (HENKEL)
[2] $C_{12\text{-}18}$-Fettalkohol (INCI-Bezeichnung: Coconut alcohol) (HENKEL)
[3] Cetylstearylalkohol mit ca. 20 EO-Einheiten (INCI-Bezeichnung: Ceteareth-20) (HENKEL)
[4] Laurylethersulfat, Natriumsalz (ca. 27,5% Aktivsubstanz; INCI-Bezeichnung: Sodium Laureth Sulfate) (HENKEL)
[5] N,N-Dimethyl-N-($C_{8\text{-}18}$-kokosamidopropyl)ammoniumacetobetain (ca. 30% Aktivsubstanz; INCI-Bezeichnung: Aqua (Water), Cocamidopropyl Betaine) (HENKEL)

Die Substanzen Hydrenol D, Lorol und Eumulgin B2 wurden bei 80°C aufgeschmolzen, mit dem 80°C heißem Wasser, enthaltend Texapon NSO und Dehyton K, vermischt und unter starkem Rühren emulgiert. Danach wurde die Emulsion unter schwachem Rühren abgekühlt.

*(b) Färbezubereitungen*

*Teilmischung B1*

| | |
|---|---|
| 4-Aminophenol | 0,27g (0,0025mol) |
| Ammoniumhydroxid[1] | ad pH 7,0 |
| Wasser | 10ml |

[1] Es wurde eine kommerzielle Ammoniumhydroxidlösung der Firma Sigma mit einem Gehalt von ca. 30% Ammoniak im Verhältnis 1:10 mit bidestilliertem Wasser vermischt.

*Teilmischung B2*

| | |
|---|---|
| 5-Amino-2-methylphenol | 0,31g (0,0025mol) |
| Ammoniumhydroxid | ad pH 7,0 |
| Wasser | 10ml |

Die Farbstoffvorprodukte wurden jeweils in 10ml Wasser gelöst und der pH-Wert der Lösungen mit Ammoniumhydroxid eingestellt.

Die Farbstoffzubereitungen (Teilmischungen B1 und B2) wurden zu 25g der bei 80°C geschmolzen Cremegrundlage (Teilmischung A) gegeben und der pH-Wert gegebenfalls mit einer wäßrigen HCl-Lösung oder mit Ammoniumhydroxid auf pH 7 eingestellt. Die Creme wurde mit Wasser auf 50g aufgefüllt und unter Rühren auf 30°C abgekühlt.

2 Herstellung der Enzymlösungen

*(a) Peroxidase-Lösung(0,41U/ml)*

Zur Herstellung der Peroxidase-Lösung wurde pulverförmige Peroxidase aus Sojabohnen (Firma Sigma; Katalog-Nummer P-1432) verwendet. Die Aktivität der Peroxidase ist derart definiert, daß eine Einheit [1U] Peroxidase 1,0 mg Purpurogallin aus Pyrogallol innerhalb von 20sec bei pH 6,0 und 20°C bildet.

Da die Aktivität bezogen auf die Masse (U/g) der Peroxidase in den Handelsprodukten chargenabhängig schwankt, ist die Herstellung der Lösung abhängig von der Aktivität der gelieferten Peroxidase.
Es wurde Peroxidase mit einer Aktivität von 73 U/mg verwendet. 1g dieser Peroxidase wurde in 1ml bidestilliertem Wasser gelöst; 28,3µl dieser Lösung wurden abgenommen und mit bidestilliertem Wasser auf 5ml aufgefüllt. Der pH-Wert der Lösung wurde mit Ammoniumhydroxid auf den Wert 7 eingestellt. Die Lösung enthält somit pro Milliliter die Menge an Peroxidase, die unter den oben genannten Bedingungen eine Aktivität von 0,41 U aufweist.

*(b) Cholin-Oxidaselösung(33,2U/ml)*

Zur Herstellung der Cholin-Oxidaselösung wurde pulverförmige Cholin-Oxidase aus *Alcaligenes species* (Firma Sigma; Katalog-Nummer C-5896) verwendet. Die Aktivität der Cholin-Oxidase ist derart definiert, daß eine Einheit [1U] Cholin-Oxidase 1µmol $H_2O_2$ durch Oxidation von 1µmol Cholin zu Betainaldehyd innerhalb einer Minute bei pH 8,0 und 37°C bildet.
Da die Aktivität bezogen auf die Masse (U/g) der Cholin-Oxidase in den Handelsprodukten chargenabhängig schwankt, ist die Herstellung der Lösung abhängig von der Aktivität der gelieferten Cholin-Oxidase.
Es wurde Cholin-Oxidase mit einer Aktivität von 14U/mg verwendet. 15mg dieser Cholin-Oxidase wurde in 5ml bidestilliertem Wasser gelöst; 3,952ml dieser Lösung wurden abgenommen und mit bidestilliertem Wasser auf 5ml aufgefüllt. Der pH-Wert der Lösung wurde mit Ammoniumhydroxid auf den Wert 7 eingestellt. Die Lösung enthält pro Milliliter die Menge an Cholin-Oxidase, die unter den oben genannten Bedingungen eine Aktivität von 33,2U aufweist.

*(c) Cholinchlorid-Lösung*

Es wurden 3,36g Cholinchlorid in 20ml bidestilliertem Wasser gelöst. Der pH-Wert der Lösung wurde mit Ammoniumhydroxid auf 7 eingestellt.

3 Inkubationsansatz

2,5ml der wäßrigen Cholinchloridlösung (gemäß 2c) wurden mit 2,5ml der wäßrigen Cholin-Oxidaselösung (33,2U/ml, gemäß 2b) vermischt und der pH-Wert mit Ammoniumhydroxid auf den Wert 7 eingestellt. Die resultierende Lösung wurde anschließend in einer Kristallisierschale für 30min bei 37°C in einer Schüttelhaube (100Upm) vorinkubiert.

4 Färbung der Haare

8g der abgekühlten Färbecreme (gemäß 1) wurden mit 4ml des Inkubationsansatzes (gemäß 3) verrührt. Anschließend wurden 4ml einer Sojabohnen-Peroxidase-Lösung (0,41U/ml) hinzugefügt. 100g des anwendungsbereiten Färbesystems enthalten die Menge Cholin-Oxidase, die unter den oben genannten Bedingungen eine Aktivität von 415U hat. Außerdem enthalten 100g des anwendungsbereiten Färbesystems die Menge an Sojabohnen-Peroxidase, die unter den oben genannten Bedingungen eine Aktivität von 10,3U aufweist.

In dieses Färbesystem wurde eine 0,5g schwere und 6 cm lange Haarsträhnc (Kerling, 80% Grauanteil) für 10min getaucht. Anschließend wurde die Färbung noch 35min auf einer Petrischale an der Luft weiterentwicklet. Das Haar wurde mit lauwarmem Wasser gespült und an der Luft getrocknet.
Die Farbe der Strähne wurde nach der Farbskala im Farbkatalog (Taschenlexikon der Farben, A. Kornerup und J.H. Wanscher, Muster-Schmidt-Verlag, 3. Unveränderte Auflage, 1981) mit 6B6, grauorange, beurteilt.

5 Vergleichstest

Zur Bestimmung des Einflusses der Enzyme wurde eine 0,5g schwere und 6cm lange Haarsträhne (Kerling, 80% Grauanteil) für 10min in die Färbecreme gemäß 1 ohne Beimischung der Enzymlösungen getaucht. Anschließend wurde die Färbung noch 35min auf einer Petrischale an der Luft weiterentwickelt. Das Haar wurde mit lauwarmem Wasser gespült und an der Luft getrocknet. Die Farbe der Haarsträhne, die sich ohne den Einfluß der Enzyme entwikkelte, wurde mit 4A5, buttergelb, beurteilt.

***Beispiel 2***

***Vergleichsversuch Cholin-Oxidasesystem und Glucose-Oxidasesystem***

1 Herstellung der Färbecreme

Es wurde die Färbecreme aus Beispiel 1 verwendet, die aber mit Ammoniumhydroxid auf einen pH-Wert von 8,3 eingestellt wurde.

2 Herstellung der Enzymlösungen

Es wurde die Peroxidase-Lösung aus Beispiel 1, Punkt 2a verwendet. Zusätzlich wurden die folgenden Lösungen hergestellt:

*(a) Pufferlösung*

Es wurden 0,01mol Tris(hydroxymethyl)-aminomethan und 0,134mol Kaliumchlorid in 950ml Wasser gelöst. Der pH-Wert dieser Lösung wurde mit 1-molarer Salzsäure auf 8,3 eingestellt und die Lösung auf einen Liter aufgefüllt.

*(b) Cholin-Oxidaselösung(33,2U/ml), Erfindung*

Zur Herstellung der Cholin-Oxidaselösung wurde pulverförmige Cholin-Oxidase aus *Alcaligenes species* (Firma Sigma; Katalog-Nummer C-5896) verwendet. Die Aktivität der Cholin-Oxidase ist derart definiert, daß eine Einheit [1U] Cholin-Oxidase 1µmol $H_2O_2$ durch Oxidation von 1µmol Cholin zu Betainaldehyd innerhalb einer Minute bei pH 8,0 und 37°C entwickelt.

Da die Aktivität bezogen auf die Masse (U/g) der Cholin-Oxidase in den Handelsprodukten chargenabhängig schwankt, ist die Herstellung der Lösung abhängig von der Aktivität der gelieferten Cholin-Oxidase.

Es wurde Cholin-Oxidase mit einer Aktivität von 14U/mg verwendet. 15mg dieser Cholin-Oxidase wurde in 5ml Pufferlösung gelöst; 3,952ml dieser Lösung wurden abgenommen und mit der Pufferlösung auf 5ml aufgefüllt. Der pH-Wert der Lösung betrug 8,3. Die Lösung enthält pro Milliliter die Menge an Cholin-Oxidase, die unter den oben genannten Bedingungen eine Aktivität von 33,2U aufweist.

*(c) Cholinchlorid-Lösung, Erfindung*

Es wurden 1,68g Cholinchlorid in 10ml Pufferlösung gelöst. Der pH-Wert der Lösung betrug 8,3.

*(d) Glucose-Oxidaselösung(33,2U/ml), Vergleichssystem*

Zur Herstellung der Glucose-Oxidaselösung wurde pulverförmige Glucose-Oxidase aus *Aspergillus niger* (Firma Sigma; Katalog-Nummer G-7141) verwendet. Die Aktivität der Glucose-Oxidase ist derart definiert, daß eine Einheit [1U] Glucose-Oxidase 1µmol $H_2O_2$ durch Oxidation von 1µmol β-D-Glucose zu D-Gluconolacton innerhalb einer Minute bei pH 5,1 und 35°C, bildet:
Da die Aktivität bezogen auf die Masse (U/g) der Glucose-Oxidase in den Handelsprodukten chargenabhängig schwankt, ist die Herstellung der Lösung abhängig von der Aktivität der gelieferten Glucose-Oxidase.
Es wurde Glucose-Oxidase mit einer Aktivität von 150U/mg verwendet. 1mg dieser Glucose-Oxidase wurde in 5ml Pufferlösung gelöst; 798µl dieser Lösung wurden abgenommen und mit Pufferlösung auf 5ml aufgefüllt. Der pH-Wert der Lösung betrug 8,3. Die Lösung enthält pro Milliliter die Menge an Glucose-Oxidase, die unter den oben genannten Bedingungen eine Aktivität von 33,2U aufweist.

*(e) Glucose-Lösung, Vergleichssystem*

Es wurden 1,376g D-(+)-Glucosemonohydrat in 10ml Pufferlösung gelöst. Der pH-Wert der Lösung betrug 8,3.

3 Inkubationsansatz

*(a) Cholin-Oxidasesystem, Erfindung*

2,5ml der wäßrigen Cholinchloridlösung wurden mit 2,5ml der wäßrigen Cholin-Oxidaselösung (33,2U/ml) vermischt. Die resultierende Lösung wurde anschließend in einer Kristallisierschale für 30min bei 37°C in einer Schüttelhaube (100Upm) vorinkubiert.

*(b) Glucose-Oxidasesystem, Vergleichssystem*

2,5ml der wäßrigen Glucoselösung wurden mit 2,5ml der wäßrigen Glucose-Oxidaselösung (33,2U/ml) vermischt. Die resultierende Lösung wurde anschließend in einer Kristallisierschale für 30min bei 37°C in einer Schüttelhaube (100Upm) vorinkubiert.

4 Färbung der Haare

Es wurden mit jedem Inkubationsansatz (Punkte 3a und 3b) Färbungen wie in Beispiel 1 beschrieben durchgeführt.

100g des anwendungsbereiten Färbesystems enthielten die Menge Cholin-Oxidase beziehungsweise Glucose-Oxidase, die unter den Punkten 2b beziehungsweise 2d genannten Bedingungen eine Aktivität von 415U hatte. Außerdem enthielten 100g des anwendungsbereiten Färbesystmes die Menge an Sojabohnen-Peroxidase, die unter den in Beispiel 1 unter Punkt 2a genannten Bedingungen eine Aktivität von 10,3U aufwies.

Die mit dem Cholin-Oxidasesystem erzielte Färbung wurde als 7A8 (brandrot/rotorange) eingestuft. Mit dem Glucose-Oxidasesystem wurde eine persischorange Färbung (6A7) erzielt.

Die Färbung, die mit dem erfindungsgemäßem Cholin-Oxidasesystem erhalten wurde, war deutlich intensiver und glänzender als die Färbung, die mit dem Glucose-Oxidasesystem erhalten wurde.

***Beispiel 3***

***Vergleichsversuch Cholin-Oxidasesystem und Xanthin-Oxidasesystem***

1 Herstellung der Färbecreme

Es wurde die Färbecreme aus Beispiel 1 verwendet, die mit Ammoniumhydroxid auf einen pH-Wert von 8,3 eingestellt wurde.

2 Herstellung der Enzymlösungen

Es wurde die Peroxidase-Lösung aus Beispiel 1 verwendet. Zusätzlich wurden die folgenden Lösungen hergestellt:

*(a) Pufferlösung*

Es wurden die Pufferlösung aus Beispiel 2, Punkt 2a verwendet.

*(b) Cholin-Oxidaselösung(8U/ml), Erfindung*

Zur Herstellung der Cholin-Oxidaselösung wurde pulverförmige Cholin-Oxidase aus *Alcaligenes species* (Firma Sigma; Katalog-Nummer C-5896) verwendet. Die Aktivität der Cholin-Oxidase ist derart definiert, daß eine Einheit [1U] Cholin-Oxidase 1μmol $H_2O_2$ durch Oxidation von 1μmol Cholin zu Betainaldehyd innerhalb einer Minute bei pH 8,0 und 37°C entwickelt.

Da die Aktivität bezogen auf die Masse (U/g) der Cholin-Oxidase in den Handelsprodukten chargenabhängig schwankt, ist die Herstellung der Lösung abhängig von der Aktivität der gelieferten Cholin-Oxidase.

Es wurde Cholin-Oxidase mit einer Aktivität von 14U/mg verwendet. 2mg dieser Cholin-Oxidase wurde in 2ml Pufferlösung gelöst; 1,714ml dieser Lösung wurden abgenommen und mit der Pufferlösung auf 3ml aufgefüllt. Der pH-Wert der Lösung betrug 8,3. Die Lösung enthält pro Milliliter die Menge an Cholin-Oxidase, die unter den oben genannten Bedingungen eine Aktivität von 8U aufweist.

*(c) Cholinchlorid-Lösung, Erfindung*

Es wurden 1,68g Cholinchlorid in 10ml Pufferlösung gelöst. Der pH-Wert der Lösung betrug 8,3.

*(d) Xanthin-Oxidaselösung(8,0U/ml), Vergleichssystem*

Zur Herstellung der Xanthin-Oxidaselösung wurde pulverförmige Xanthin-Oxidase aus Mikroorganismen (Firma Sigma; Katalog-Nummer X-2252) verwendet. Die Aktivität der Xanthin-Oxidase ist derart definiert, daß eine Einheit [1U] Xanthin-Oxidase 1μmol Xanthin zu Harnsäure innerhalb einer Minute bei pH 7,5 und 25°C oxidiert.
Da die Aktivität bezogen auf die Masse (U/g) der Xanthin-Oxidase in den Handelsprodukten chargenabhängig schwankt, ist die Herstellung der Lösung abhängig von der Aktivität der gelieferten Xanthin-Oxidase.
Es wurde Xanthin-Oxidase mit einer Aktivität von 8,6U/mg verwendet. 3mg dieser Xanthin-Oxidase wurde in 1ml Pufferlösung gelöst; 930,2μl dieser Lösung wurden abgenommen und mit Pufferlösung auf 3ml aufgefüllt. Der pH-Wert der Lösung betrug 8,3. Die Lösung enthält pro Milliliter die Menge an Xanthin-Oxidase, die unter den oben genannten Bedingungen eine Aktivität von 8U aufweist.

*(e) Xanthin-Lösung, Vergleichssystem*

Es wurden 0,0018g Xanthin in 10ml Pufferlösung gelöst. Der pH-Wert der Lösung betrug 8,3.

3 Inkubationsansatz

*(a) Cholin-Oxidasesystem, Erfindung*

2,5ml der wäßrigen Cholinchloridlösung wurden mit 2,5ml der wäßrigen Cholin-Oxidaselösung (8U/ml) vermischt. Die resultierende Lösung wurde anschließend in einer Kristallisierschale für 30min bei 37°C in einer Schüttelhaube (100Upm) vorinkubiert.

*(b) Xanthin-Oxidasesystem, Vergleichssystem*

2,5ml der wäßrigen Xanthinlösung wurden mit 2,5ml der wäßrigen Xanthin-Oxidaselösung (8U/ml) vermischt. Die resultierende Lösung wurde anschließend in einer Kristallisierschale für 30min bei 37°C in einer Schüttelhaube (100Upm) vorinkubiert.

4 Färbung der Haare

Es wurden mit jeden Inkubationsansatz Färbungen wie in Beispiel 1 beschrieben durchgeführt.
100g des anwendungsbereiten Färbesystems enthielten die Menge Cholin-Oxidase beziehungsweise Xanthin-Oxidase, die unter den in Punkt 2b beziehungsweise 2d genannten Bedingungen eine Aktivität von 100U hatte. Außerdem enthielten 100g des anwendungsbereiten Färbesystmes die Menge an Sojabohnen-Peroxidase, die unter den in Beispiel 1, Punkt 2a genannten Bedingungen eine Aktivität von 10,3U aufwies.
Die Bewertung der Farbtöne wurde analog zu Beispiel 1 vorgenommen.
Die mit dem Cholin-Oxidasesystem erzielte Färbung wurde als 6A7 (persischorange) bewertet. Mit dem Xanthin-Oxidasesystem wurde eine buttergelbe Färbung (4A5) erzielt. Die Färbung, die mit dem erfindungsgemäßem Cholin-Oxidasesystem erhalten wurde, ist deutlich intensiver und glänzender als die Färbung, die mit dem Xanthin-Oxidasesystem erhalten wurde.

***Weitere Beispiele***

Weiterhin wurden die folgenden Ausfärbungen analog zu den Vorschriften des Beispiel 1 durchgeführt:

1 Ausfärbungen mit 1-Methyl-2,5-diaminobenzol als Entwickler

| Kuppler Farbton | |
|---|---|
| 1,3-Bis-(2,4-diaminophenoxy)-propan + 4HCl | 20F4<br>tintenblau |
| 2,4-Diaminophenoxyethanol + 2 HCl | 20F5<br>schwarzblau |
| 3-Amino-6-methoxy-2-methylaminopyridin + 2 HCl | 4F3<br>olivbraun |
| 1-Naphthol | 6E3<br>graubraun |
| 2,4-Dichlor-3-aminophenol | 13F2<br>purpurgrau |
| 2-Amino-3-hydroxypyridin | 8E3<br>graubraun |
| 5-Amino-4-chlor-2-methylphenol + HCl | 9D5<br>bois-de-rose |
| 5-Amino-2-methylphenol | 10E4<br>violettbraun |
| Resorcin | 4B5<br>buttergelb |
| 3-Aminophenol | 4B5<br>buttergelb |

2 Ausfärbungen mit 1,4-Diaminobenzol als Entwickler

| Kuppler | Farbton |
|---|---|
| 2,4-Diaminophenoxyethanol + 2 HCl | 19F8<br>schwarzblau |
| 4-Chlorresorcin | 4B4<br>champagner |
| 2-Methyl-5-Aminophenol | 11C5<br>graurot |
| 3-Aminophenol | 4B4<br>champagner |
| 2,7-Dihydroxynaphthalin | 4B4<br>champagner |

3 Ausfärbungen mit 2,4,5,6-Tetraaminopyridin· $H_2SO_4$ als Entwickler

| Kuppler | Farbton |
|---|---|
| 2-Methylresorcin | 6A5<br>lachsrot |

(fortgesetzt)

| Kuppler | Farbton |
|---|---|
| 2,7-Dihydroxynaphthalin | 4B5 weizengold |
| 1-Methyl-2,6-bis-(2-hydroxyethylamino-)benzol | 12B8 rosenrot |

4 Ausfärbungen mit 4-Aminophenol als Entwickler

| Kuppler | Farbton |
|---|---|
| 5-Amino-2-methylphenol | 6B6 grauorange |
| 1-Naphthol | 6B3 hautfarbig |
| 1,3-Bis-(2,4-diaminophenoxy)-propan + 4HCl | 8D4 rotbraun |
| 6-Hydroxyindol | 5C4 goldblond |

5 Ausfärbungen mit 4-Hydroxy-2,5,6-triaminopyrimidin als Entwickler

| Kuppler | Farbton |
|---|---|
| 2,4-Diaminophenoxyethanol + 2 HCl | 16F6 dunkelviolett |
| 1,3-Bis-(2,4-diaminophenoxy)-propan + 4HCl | 15F6 dunkelviolett |
| Resorcin | 7C5 braunorange |
| 2-Methylresorcin (3-fach konzentriert) | 8B5 graurot |

6 Ausfärbungen mit N,N-Bis-(β-hydroxyethyl)-1,4-diaminobenzol als Entwickler

| Kuppler | Farbton |
|---|---|
| 2,6-Dimethoxy-3,5-diaminopyridin | 23F8 dunkelblau |
| 1-Methoxy-2-amino-4-(β-hydroxyethylamino)-benzol | 21F8 dunkelblau |
| 5-Amino-4-chlor-2-methylphenol | 17F7 dunkelviolett |
| 3,4-Methylendioxyanilin + HCl | 6E4 braun |

7 Ausfärbungen mit 1-(2'-Hydroxyethyl)-2,5-diaminobenzol als Entwickler

| Kuppler | Farbton |
|---|---|
| 2-Chlor-6-methyl-3-aminophenol | 14F5 dunkelpurpur |
| 2,4-Diaminophenoxyethynol + 2 HCl | 20F5 schwarzblau |
| 2-Methylresorcin | 5C4 goldblond |
| 3,4-Methylendioxyanilin + HCl | 5C3 rotblond |
| 2-Aminophenol | 4C4 z blond |

8 Ausfärbungen mit 3-Methyl-4-aminophenol als Entwickler

| Kuppler | Farbton |
|---|---|
| 3-Aminophenol | 4B4 champagner |
| 3-Amino-6-methoxy-2-methylaminopyridin | 4B6 graugelb |
| 2-Amino-3-hydroxypyridin | 5C4 goldblond |
| 1-Methoxy-2-amino-4-(β-hydroxyethylamino)-benzol | 9E4 rotbraun |
| 5-Amino-2-methylphenol | 6B7 möhrenrot |

9 Ausfärbungen mit 4-Amino-2-aminomethylphenol · 2 HCl als Entwickler

| Kuppler | Farbton |
|---|---|
| 2-Chlor-6-methyl-3-aminophenol | 7B7 rotorange |
| 2,4-Diaminophenoxyethanol + 2 HCl | 9D5 bois-de-rose |
| 2,6-Dimethoxy-3,5-diaminopyridin | 27F4 dunkelgrün |
| 2-Aminophenol | 4A5 buttergelb |

10 Ausfärbungen mit 1,8-Bis-(2,5-Diaminophenoxy)-3,6-dioxa-octan als Entwickler

| Kuppler | Farbton |
|---|---|
| 3-Amino-6-methoxy-2-methylaminopyridin | 5E3 mausgrau |
| 1,3-Bis-(2,4-diaminophenoxy)propan + 4 HCl | 20E4 türkischblau |

## Patentansprüche

1. Mittel zum Färben keratinischer Fasern, enthaltend mindestens ein Farbstoffvorprodukt, Cholin-Oxidase in Kombination mit Cholin als Substrat sowie mindestens eine Peroxidase, **dadurch gekennzeichnet, dass** die Cholin-Oxidase in Mengen von 1-50 000U pro 100g Färbezubereitung, das Substrat Cholin in Mengen von 1-5 Gew.-%, bezogen auf die gesamte Färbezubereitung, und die Peroxidase in einer Menge von 1-100 000U, bezogen auf 100g Färbezubereitung, eingesetzt werden.

2. Mittel nach Anspruch 1, **dadurch gekennzeichnet, dass** Cholin-Oxidase, die von *Alcaligenes species* und *Arthrobacter globiformis* produziert wird, eingesetzt wird.

3. Mittel nach einem der Ansprüche 1 oder 2, **dadurch gekennzeichnet, dass** das Farbstoffvorprodukt ein Oxidationsfarbstoffvorprodukt vom Typ Entwickler ist.

4. Mittel nach einem der Ansprüche 1 bis 3, **dadurch gekennzeichnet, dass** die Entwicklerkomponente ausgewählt ist aus p-Phenylendiamin, p-Toluylendiamin, p-Aminophenol, 1-(2'-Hydroxyethyl)-2,5-diaminobenzol, 4-Amino-3-methylphenol, 2,4,5,6-Tetraaminopyrimidin, 2-Hydroxy-4,5,6-triaminopyrimidin, 4-Hydroxy-2,5,6-tri-aminopyrimidin, 4,5-Diamino-1-(2-hydroxyethyl)-pyrazol, 2-Aminomethyl-4-amino-phenol, N,N-Bis-(2-hydroxyethyl)-p-phenylendiamin, Bis-(2-hydroxy-5-aminophenyl)-methan und o-Aminophenol.

5. Mittel nach einem der Ansprüche 1 bis 4, **dadurch gekennzeichnet, dass** es weiterhin mindestens eine Kupplerkomponente ausgewählt aus der Gruppe, gebildet von 1-Naphthol, 1,5-, 2,7- und 1,7-Dihydroxynaphthalin, 5-Amino-2-methylphenol, Resorcin, 1,3-Bis-(2,4-diaminophenoxy)-propan, 2,4-Dichlor-3-aminophenol, 4-Chlorresorcin, 2-Amino-3-hydroxypyridin, 2,6-Dimethoxy-3,5-diaminopyridin, 2-Chlor-6-methyl-3-aminophenol, 2-Methyl-4-chlor-5-amino-phenol, 2-Methylresorcin, 5-Methylresorcin, 2,5-Dimethylresorcin, 2,6-Bis-(2-hydroxyethylamino)-toluol, 2,6-Dihydroxy-3,4-diaminopyridin, 3-Amino-2-methylamino-6-methoxypyridin, 2,4-Diaminophenoxyethanol, 1-Methoxy-2-amino-4-(2-hydroxyethylamino)-benzol, 6-Methyl-1,2,3,4-tetrahydro-chinoxalin, 3,4-Methylendioxyanilin, m-Aminophenol, o-Aminophenol und 2-Chlorresorcin enthält.

6. Mittel nach einem der Ansprüche 1 bis 5, **dadurch gekennzeichnet, dass** es mindestens eine Entwickler-/Kupplerkombinationen ausgewählt aus

   - 4-Aminophenol/5-Amino-2-methylphenol
   - 1-Methyl-2,5-diaminobenzol/2,4-Diaminophenoxyethanol
   - 4-Amino-2-aminomethylphenol/2-Chlor-6-methyl-3-aminophenol
   - 4-Amino-2-aminomethylphenol/ 2,4-Diaminophenoxyethanol
   - 1-Methyl-2,5-diaminobenzol/5-Amino-2-methylphenol
   - 1-Methyl-2,5-diaminobenzol/1,3-Bis-(2,4-diaminophenoxy)-propan
   - 4-Hydroxy-2,5,6-triaminopyrimidin/2-Methylresorcin
   - 1-(β-Hydroxyethyl)-2,5-diaminobenzol/2,4-Diaminophenoxyethanol
   - N,N-Bis-(β-hydroxyethyl)-1,4-diaminobenzol/1-Methoxy-2-amino-4-(2-hydroxyethyl-amino)-benzol

   enthält.

7. Mittel nach einem der Ansprüche 1 oder 2, **dadurch gekennzeichnet, dass** das Farbstoffvorprodukt ein Derivat des 5,6-Dihydroxyindolins der Formel (Ia) ist,

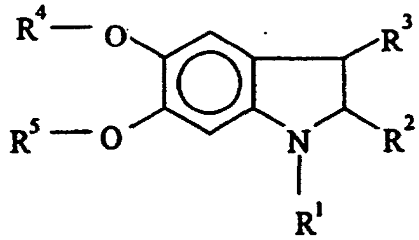

(Ia)

in der unabhängig voneinander
$R^1$ steht für Wasserstoff, eine $C_1$- bis $C_4$-Alkylgruppe oder eine $C_1$- bis $C_4$-Hydroxyalkylgruppe,
$R^2$ steht für Wasserstoff oder eine -COOH-Gruppe, wobei die -COOH-Gruppe auch als Salz mit einem physiologisch verträglichen Kation vorliegen kann,
$R^3$ steht für Wasserstoff oder eine $C_1$- bis $C_4$-Alkylgruppe,
$R^4$ steht für Wasserstoff eine $C_1$- bis $C_4$-Alkylgruppe oder eine Gruppe -CO-$R^6$, in der $R^6$ steht für eine $C_1$- bis $C_4$-Alkylgruppe, und
$R^5$ steht für eine der unter $R^4$ genannten Gruppen,
oder ein physiologisch verträgliches Salz dieser Verbindungen mit einer organischen oder anorganischen Säure.

**8.** Mittel nach einem der Ansprüche 1 oder 2, **dadurch gekennzeichnet, dass** das Farbstoffvorprodukt ein Derivat des 5,6-Dihydroxyindols der Formel (Ib) ist,

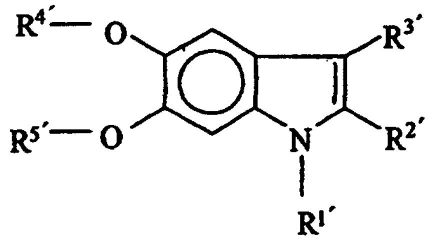

(Ib)

in der unabhängig voneinander
$R^{1\cdot}$ steht für Wasserstoff, eine $C_1$- bis $C_4$-Alkylgruppe oder eine $C_1$- bis $C_4$-Hydroxyalkylgruppe,
$R^{2\cdot}$ steht für Wasserstoff oder eine -COOH-Gruppe, wobei die -COOH-Gruppe auch als Salz mit einem physiologisch verträglichen Kation vorliegen kann,
$R^{3\cdot}$ steht für Wasserstoff oder eine $C_1$- bis $C_4$-Alkylgruppe,
$R^{4\cdot}$ steht für Wasserstoff, eine $C_1$- bis $C_4$-Alkylgruppe oder eine Gruppe -CO-$R^{6\cdot}$, in der $R^{6\cdot}$ steht für eine $C_1$- bis $C_4$-Alkylgruppe, und
$R^{5\cdot}$ steht für eine der unter $R^{4\cdot}$ genannten Gruppen,
oder ein physiologisch verträgliches Salz dieser Verbindungen mit einer organischen oder anorganischen Säure.

**9.** Mittel nach einem der Ansprüche 1 bis 8, **dadurch gekennzeichnet, dass** die Peroxidase aus Pflanzen oder Pilzen stammt.

**10.** Mittel nach Anspruch 9, **dadurch gekennzeichnet, dass** es eine Sojabohnen-Peroxidase enthält.

**11.** Mittel nach einem der Ansprüche 1 bis 10, **dadurch gekennzeichnet, dass** es einen pH-Wert von 7 bis 10 aufweist.

**12.** Mittel nach Anspruch 11, **dadurch gekennzeichnet, dass** einen pH-Wert von etwa 8,3 hat.

**13.** Mittel nach einem der Ansprüche 1 bis 12, **dadurch gekennzeichnet, dass** es weiterhin mindestens ein Tensid enthält.

**14.** Mittel nach Anspruch 13, **dadurch gekennzeichnet, dass** es ein nichtionisches oder ein amphoteres Tensid enthält.

**15.** Mittel nach einem der Ansprüche 13 oder 14, **dadurch gekennzeichnet, dass** es eine Tensidkombination aus mindestens einem anionischen Tensid mit mindestens einem amphoteren Tensid und/oder mit mindestens einem nichtionischen Tensid enthält.

**16.** Verfahren zum Färben keratinischer Fasern, **dadurch gekennzeichnet, dass** ein Mittel nach einem der Ansprüche 1 bis 15 eingesetzt wird.

**17.** Verfahren nach Anspruch 16, **dadurch gekennzeichnet, dass** das Oxidasesystem vorher für 30 Minuten bei 37°C inkubiert wird.

**18.** Verwendung eines der Mittel nach einem der Ansprüche 1 bis 15 zum Färben keratinischer Fasern.

**Claims**

**1.** A composition for colouring keratin fibres containing at least one dye precursor, choline oxidase in combination with choline as substrate and at least one peroxidase, **characterized in that** the choline oxidase is used in quantities of 1 to 50,000 U per 100 g colouring preparation, the substrate choline is used in quantities of 1 to 5% by weight, based on the colouring preparation as a whole, and the peroxidase is used in a quantity of 1 to 100,000 U, based on 100 g of colouring preparation.

**2.** A composition as claimed in claim 1, **characterized in that** choline oxidase produced by *Alcaligenes species* and *Arthrobacter globiformis* is used.

**3.** A composition as claimed in claim 1 or 2, **characterized in that** the dye precursor is an oxidation dye precursor of the primary intermediate type.

**4.** A composition as claimed in any of claims 1 to 3, **characterized in that** the primary intermediate is selected from p-phenylenediamine, p-toluylenediamine, p-aminophenol, 1-(2'-hydroxyethyl)-2,5-diaminobenzene, 4-amino-3-methylphenol, 2,4,5,6-tetraaminopyrimidine, 2-hydroxy-4,5,6-triaminopyrimidine, 4-hydroxy-2,5,6-triaminopyrimidine, 4,5-diamino-1-(2-hydroxyethyl)-pyrazole, 2-aminomethyl-4-aminopenol, N,N-bis-(2-hydroxyethyl)-p-phenylenediamine, bis-(2-hydroxy-5-aminophenyl)-methane and o-aminophenol.

**5.** A composition as claimed in any of claims 1 to 4, **characterized in that** it also contains at least one secondary intermediate selected from the group consisting of 1-naphthol, 1,5-, 2,7- and 1,7-dihydroxynaphthalene, 5-amino-2-methylphenol, resorcinol, 1,3-bis-(2,4-diaminophenoxy)-propane, 2,4-dichloro-3-aminophenol, 4-chlororesorcinol, 2-amino-3-hydroxypyridine, 2,6-dimethoxy-3,5-diaminopyridine, 2-chloro-6-methyl-3-aminophenol, 2-methyl-4-chloro-5-aminophenol, 2-methyl resorcinol, 5-methyl resorcinol, 2,5-dimethyl resorcinol, 2,6-bis-(2-hydroxyethylamino)-toluene, 2,6-dihydroxy-3,4-diaminopyridine, 3-amino-2-methylamino-6-methoxypyridine, 2,4-diaminophenoxy ethanol, 1-methoxy-2-amino-4-(2-hydroxyethylamino)-benzene, 6-methyl-1,2,3,4-tetrahydroquinoxaline, 3,4-methylenedioxy aniline, m-aminophenol, o-aminophenol and 2-chlororesorcinol.

**6.** A composition as claimed in any of claims 1 to 5, **characterized in that** it contains at least one primary intermediate/secondary intermediate combination selected from

- 4-aminophenol/5-amino-2-methylphenol
- 1-methyl-2,5-diaminobenzene/2,4-diaminophenoxyethanol
- 4-amino-2-aminomethylphenol/2-chloro-6-methyl-3-aminophenol
- 4-amino-2-aminomethylphenol/2,4-diaminophenoxy ethanol
- 1-methyl-2,5-diaminobenzene/5-amino-2-methylphenol
- 1-methyl-2,5-diaminobenzene/1,3-bis-(2,4-diaminophenoxy)-propane
- 4-hydroxy-2,5,6-triaminopyridine/2-methylresorcinol
- 1-(β-hydroxyethyl)-2,5-diaminobenzene/2,4-diaminophenoxy ethanol
- N,N-bis-(β-hydroxyethyl)-1,4-diaminobenzene/1-methoxy-2-amino-4-(2-hydroxyethylamino)-benzene.

**7.** A composition as claimed in claim 1 or 2, **characterized in that** the dye precursor is a derivative of 5,6-dihydroxyindoline corresponding to formula (Ia):

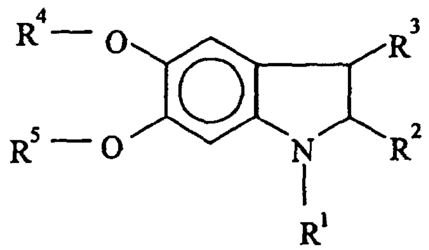

(Ia)

in which - independently of one another - $R^1$ is hydrogen, a $C_{1-4}$ alkyl group or a $C_{1-4}$ hydroxyalkyl group, $R^2$ is hydrogen or a -COOH group, the -COOH group optionally being present as a salt with a physiologically compatible cation, $R^3$ is hydrogen or a $C_{1-4}$ alkyl group, $R^4$ is hydrogen, a $C_{1-4}$ alkyl group or a group -CO-$R^6$, where $R^6$ is a $C_{1-4}$ alkyl group, and $R^5$ is one of the groups mentioned for $R^4$,
or a physiologically compatible salt of these compounds with an organic or inorganic acid.

**8.** A composition as claimed in claim 1 or 2, **characterized in that** the dye precursor is a derivative of 5,6-dihydroxyindole corresponding to formula (Ib):

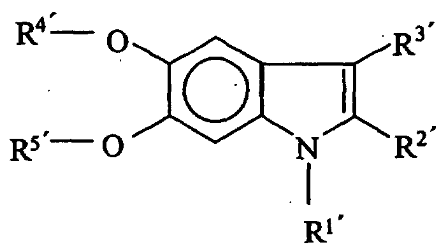

(Ib)

in which - independently of one another - $R^{1'}$ is hydrogen, a $C_{1-4}$ alkyl group or a $C_{1-4}$ hydroxyalkyl group, $R^{2'}$ is hydrogen or a -COOH group, the - COOH group optionally being present as a salt with a physiologically compatible cation, $R^{3'}$ is hydrogen or a $C_{1-4}$ alkyl group, $R^{4'}$ is hydrogen, a $C_{1-4}$ alkyl group or a group -CO-$R^{6'}$, where $R^{6'}$ is a $C_{1-4}$ alkyl group, and $R^{5'}$ is one of the groups mentioned for $R^{4'}$,
or a physiologically compatible salt of these compounds with an organic or inorganic acid.

**9.** A composition as claimed in any of claims 1 to 8, **characterized in that** the peroxidase comes from plants or fungi.

**10.** A composition as claimed in claim 9, **characterized in that** it contains a soya bean peroxidase.

**11.** A composition as claimed in any of claims 1 to 10, **characterized in that** it has a pH value of 7 to 10.

**12.** A composition as claimed in claim 11, **characterized in that** it has a pH value of about 8.3.

**13.** A composition as claimed in any of claims 1 to 12, **characterized in that** it also contains at least one surfactant.

**14.** A composition as claimed in claim 1, **characterized in that** it contains a nonionic surfactant or an amphoteric surfactant.

**15.** A composition as claimed in claim 13 or 14, **characterized in that** it contains a surfactant combination of at least one anionic surfactant and at least one amphoteric surfactant and/or at least one nonionic surfactant.

**16.** A process for colouring keratin fibres, **characterized in that** the composition claimed in any of claims 1 to 15 is used.

**17.** A process as claimed in claim 16, **characterized in that** the oxidase system is preincubated for 30 minutes at 37°C.

**18.** The use of one of the compositions claimed in any of claims 1 to 15 for colouring keratin fibres.

**Revendications**

1. Agent pour la coloration de fibres kératiniques, contenant au moins un précurseur de colorant, de la choline-oxydase en combinaison avec de la choline à titre de substrat, ainsi qu'au moins une peroxydase, **caractérisé en ce qu'**on met en oeuvre la choline-oxydase dans des quantités de 1 à 50.000 U par 100 g de préparation de coloration, le substrat de choline dans des quantités de 1 à 5 % en poids, rapportés à la préparation de coloration totale et la peroxydase en une quantité de 1 à 100.000 U rapportée à 100 g de préparation de coloration.

2. Agent selon la revendication 1, **caractérisé en ce qu'**on met en oeuvre de la choline-oxydase que l'on obtient à partir de *Alcaligenes species* et de *Arthrobacter globiformis*.

3. Agent selon l'une quelconque des revendications 1 ou 2, **caractérisé en ce que** le précurseur de colorant est un précurseur de colorant d'oxydation de type révélateur.

4. Agent selon l'une quelconque des revendications 1 à 3, **caractérisé en ce que** le composant de révélateur est choisi parmi le groupe comprenant la p-phénylènediamine, la p-toluylènediamine, le p-aminophénol, le 1-(2'-hydroxyéthyl)-2,5-diaminobenzène, le 4-amino-3-méthylphénol, la 2,4,5,6-tétraaminopyrimidine, la 2-hydroxy-4,5,6-triaminopyrimidine, la 4-hydroxy-2,5,6-triamino-pyrimidine, le 4,5-diamino-1-(2-hydroxyéthyl)-pyrazote, le 2-aminométhyl-4-aminophénol, la N,N-bis-(2-hydroxyéthyl)-p-phénylènediamine, le bis-(2-hydroxy-5-aminophényl)-méthane et le o-aminophénol.

5. Agent selon l'une quelconque des revendications 1 à 4, **caractérisé en ce qu'**il contient en outre au moins un composant couplant choisi parmi le groupe formé par le 1-naphtol, le 1,5-, le 2,7-et le 1,7-dihydroxynaphtalène, le 5-amino-2-méthylphénol, le résorcinol, le 1,3-bis-(2,4-diaminophénoxy)-propane, le 2,4-dichloro-3-amino)-phénol, le 4-chlororésorcinol, la 2-amino-3-hydroxypyridine, la 2,6-diméthoxy-3,5-diaminopyridine, le 2-chloro-6-méthyl-3-aminophénol, le 2-méthyl-4-chloro-5-amino-phénol, le 2-méthylrésorcinol, le 5-méthylrésorcinol, le 2,5-diméthylrésorcinol, le 2,6-bis-(2-hydroxy-éthylamino)-toluène, la 2,6-dihydroxy-3,4-diaminopyridine, la 3-amino-2-méthylamino-6-méthoxypyridine, le 2,4-diaminophénoxy-éthanol; le 1-méthoxy-2-amino-4-(2-hydroxyéthylamino)-benzène, la 6-méthyl-1, 2,3,4-tétrahydroquinoxaline, la 3,4-méthylénedioxy-aniline, le m-aminophénol, le o-aminophénol et le 2-chlororésorcinol.

6. Agent selon l'une quelconque des revendications 1 à 5, **caractérisé en ce qu'**il contient une combinaison révélateur/couplant choisie parmi le groupe comprenant

   - 4-aminophénol/5-amino-2-méthylphénol
   - 1-méthyl-2,5-diaminobenzène/2,4-diaminophénoxyéthanol
   - 4-amino-2-aminométhylphénol/2-chloro-6-méthyl-3-amino-phénol
   - 4-amino-2-aminométhylphénol/2,4-diaminophénoxyéthanol
   - 1-méthyl-2,5-diaminobenzène/5-amino-2-méthylphénol
   - 1-méthyl-2,5-diaminobenzène/1,3-bis-(2,4-diamino-phénoxy)-propane
   - 4-hydroxy-2,5,6-triaminopyrimidine/2-méthylrésorcinol
   - 1-(β-hydroxyéthyl)-2,5-diaminobenzène/2,4-diaminophénoxyéthanol
   - N,N-bis-(β-hydroxyéthyl)-1,4-diaminobenzène/1-méthoxy-2-amino-4-(2-hydroxyéthyl-amino)-benzène.

7. Agent selon l'une quelconque des revendications 1 ou 2, **caractérisé en ce que** le précurseur de colorant est un dérivé de la 5,6-dihydroxyindoline répondant à la formule (Ia)

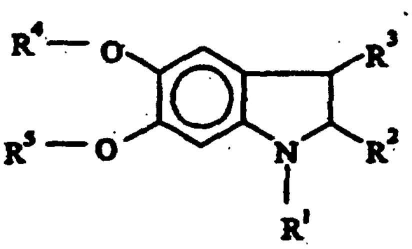

(Ia)

dans laquelle, indépendamment les uns des autres, R' représente un atome d'hydrogène, un groupe alkyle en $C_1$-$C_4$ ou un groupe hydroxyalkyle en $C_1$-$C_4$ ; $R^2$ représente un atome d'hydrogène, ou un groupe -COOH, le groupe - COOH pouvant également être présent sous la forme d'un sel contenant un cation physiologiquement acceptable ; $R^3$ représente un atome d'hydrogène ou un groupe alkyle en $C_1$-$C_4$ ; $R^4$ représente un atome d'hydrogène, un groupe alkyle en $C_1$-$C_4$ ou un groupe -CO-$R^6$ dans lequel $R^6$ représente un groupe alkyle en $C_1$-$C_4$ ; et $R^5$ représente un des groupes mentionnés sous $R^4$ ; ou encore un sel physiologiquement acceptable de ces composés avec un acide organique ou inorganique.

8. Agent selon l'une quelconque des revendications 1 ou 2, **caractérisé en ce que** le précurseur de colorant est un dérivé du 5,6-dihydroxyindole répondant à la formule (Ib)

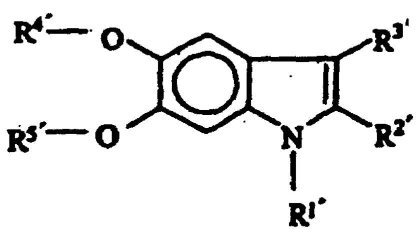

(Ib)

dans laquelle, indépendamment les uns des autres, $R^{1'}$ représente un atome d'hydrogène, un groupe alkyle en $C_1$-$C_4$ ou un groupe hydroxyalkyle en $C_1$-$C_4$ ; $R^{2'}$ représente un atome d'hydrogène, ou un groupe -COOH, le groupe - COOH pouvant également être présent sous la forme d'un sel contenant un cation physiologiquement acceptable ; $R^{3'}$ représente un atome d'hydrogène ou un groupe alkyle en $C_1$-$C_4$ ; $R^{4'}$ représente un atome d'hydrogène, un groupe alkyle en $C_1$-$C_4$ ou un groupe -CO-$R^{6'}$ dans lequel $R^{6'}$ représente un groupe alkyle en $C_1$-$C_4$ ;et $R^{5'}$ représente un des groupes mentionnés sous $R^{4'}$ ; ou encore un sel physiologiquement acceptable de ces composés avec un acide organique ou inorganique.

9. Agent selon l'une quelconque des revendications 1 à 8, **caractérisé en ce que** la peroxydase dérive de plantes ou de champignons.

10. Agent selon la revendication 9, **caractérisé en ce qu'**il contient une peroxydase de soja.

11. Agent selon l'une quelconque des revendications 1 à 10, **caractérisé en ce qu'**il présente une valeur de pH de 7 à 10.

12. Agent selon la revendication 11, **caractérisé en ce qu'**il possède une valeur de pH d'environ 8,3.

13. Agent selon l'une quelconque des revendications 1 à 12, **caractérisé en ce qu'**il contient en outre au moins un agent tensioactif.

14. Agent selon la revendication 13, **caractérisé en ce qu'**il contient un agent tensioactif non ionique ou un agent tensioactif amphotère.

15. Agent selon l'une quelconque des revendications 13 ou 14, **caractérisé en ce qu'**il contient une combinaison d'agents tensioactifs, d'au moins un agent tensioactif anionique avec au moins un agent tensioactif amphotère et/ou avec au moins un agent tensioactif non ionique.

16. Procédé pour la coloration de fibres kératiniques, **caractérisé en ce qu'**on met en oeuvre un agent selon l'une quelconque des revendications 1 à 15:

17. Procédé selon la revendication 16, **caractérisé en ce qu'**on incube le système d'oxydase au préalable pendant 30 minutes à 37 °C.

**18.** Utilisation d'un des agents selon l'une quelconque des revendications 1 à 15 pour la coloration de fibres kératiniques.